(19) Europäisches Patentamt
European Patent Office
Office européen des brevets

(11) Numéro de publication: **0 256 907 B1**

(12) **FASCICULE DE BREVET EUROPEEN**

(45) Date de publication de fascicule du brevet: **16.10.91**

(51) Int. Cl.[5]: **A61K 35/78**, A61K 39/395, A61K 37/02

(21) Numéro de dépôt: **87401656.1**

(22) Date de dépôt: **15.07.87**

(54) **Inhibiteur de la synthèse protéique, procédé d'isolement, utilisation et compositions pharmaceutiques en contenant.**

(30) Priorité: **15.07.86 FR 8610296**

(43) Date de publication de la demande:
**24.02.88 Bulletin 88/08**

(45) Mention de la délivrance du brevet:
**16.10.91 Bulletin 91/42**

(84) Etats contractants désignés:
**AT BE CH DE ES FR GB GR IT LI LU NL SE**

(56) Documents cités:
**EP-A- 0 169 111**
**FR-A- 2 437 213**

**CHEMICAL ABSTRACTS, vol. 104, no. 19, 12 mai 1986, page 384, résumé no. 165373c, Columbus, Ohio, US; Y. WANG: "Chemistry of trichosanthin, a new biologically active plant protein", & ADV. CHIN. MED. MATER. RES. INT. SYMP. 1984, 289(95)**

**IDEM**

(73) Titulaire: **SANOFI S.A.**
**40, Avenue George V**
**F-75008 Paris(FR)**

(72) Inventeur: **Barbieri, Luigi**
**Via San Giorgio 2616 B**
**I-40024 Castel San Pietro Terme Bologna(IT)**
Inventeur: **Casellas, Pierre**
**Rue de l'Aiguelongue La Colombe No. 9**
**F-34100 Montpellier(FR)**
Inventeur: **Stirpe, Fiorenzo**
**Via San Petronio Vecchio**
**I-40125 Bologna(IT)**

(74) Mandataire: **Gillard, Marie-Louise et al**
**Cabinet Beau de Loménie 55, Rue d'Amsterdam**
**F-75008 Paris(FR)**

**EP 0 256 907 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

NATURE, vol. 321, 29 mai 1986, pages 477-478; ZHANG XUEJUN et al.: "Homology of trichosanthin and ricin A chain"

IDEM

CHEMICAL ABSTRACTS, vol. 97, no. 19, 8 novembre 1982, page 280, résumé no. 158247y, Columbus, Ohio, US; S. JIN et al.: "Chemistry of trichosanthin. I. Physical and chemical properties of crystalline trichosanthin"

## Description

La présente invention se rapporte à un nouvel inhibiteur de la synthèse protéique ainsi qu'à des méthodes pour son isolement, à son utilisation et aux compositions pharmaceutiques en contenant.

Des protéines qui inhibent la synthèse protéique dans les systèmes eucaryotes ont été trouvées dans les graines ou d'autres parties d'un certain nombre de plantes. Parmi ces protéines, la ricine, l'abrine et la modécine inhibent la synthèse protéique aussi bien dans les systèmes cellulaires que dans les systèmes acellulaires et sont de ce fait hautement toxiques chez l'animal. D'autres, au contraire, efficaces sur les systèmes acellulaires seulement, sont peu ou pas toxiques chez l'animal peut-être parce qu'elles ne peuvent pénétrer dans les cellules.

D'une façon générale, ces protéines inactivent de façon irréversible la sous-unité ribosomale 60 S, qui se trouve dans l'impossibilité de réagir avec le facteur d'élongation 2. Par suite, elles présentent un intérêt pour l'étude de la synthèse protéique et il a été proposé aussi de les utiliser pour combattre les tumeurs.

Une protéine de ce type sera désignée ci-après par le terme "Ribosome inactivating Protein" (RIP). La RIP la mieux connue est la Ricine qui est obtenue par extraction des graines de Ricin. Une autre RIP, la gélonine, a été décrite récemment et une revue sur les RIP a été publiée dans FEBS Letters, 1986, 195 (1,2), pages 1-8.

Il a récemment été décrit une protéine extraite des racines de Trichosanthes kirilowii, la trichosanthine (Nature, 1986, 321, 477-478) montrant un certain degré d'homologie de séquence avec la sous unité A de la ricine D.

On a maintenant trouvé que l'on peut extraire des graines de Trichosanthes kirilowii, une nouvelle RIP exceptionnellement puissante, différente de la trichosanthine. Cette nouvelle RIP sera ci-après désignée "trichokirine".

Ainsi, la présente invention concerne, selon un de ses aspects la nouvelle RIP dénommée trichokirine ayant les caractéristiques suivantes :

C'est une glycoprotéine :

- ayant un poids moléculaire de 28.000 ± 3.000, déterminé par électrophorèse sur gel de polyacrylamide en présence de SDS (sodium dodécyl sulfate),
- de point isoélectrique ≧ 9,
- de contenu en sucres neutres : 1,1 à 1,5 % en poids dont 0,3 à 1,2 % de mannose.
- de compositions en acides aminés : en nombre résidus par mole de protéine ± 20 %.

Lys : 17,3 ; His : 1,1 ; Arg : 6,7 ; Asx : 22,8 ;
Thr : 18,9 ; Ser : 23,5 ; Glx : 21,6 ; Pro : 8,0 ;
Gly : 16,0 ; Ala : 21,1 ; 1/2 Cys : 1,9 ;
Val : 12,5 ; Met : 3,05 ; Ile : 15,8 ; Leu : 24,3 ;
Tyr : 12,1 ; Phe : 10,1 ; Trp : n.d..

dans ce qui précède, Asx représente l'ensemble des résidus d'acide aspartique et d'asparagine et Glx l'ensemble des résidus d'acide glutamique et de glutamine.

1/2 Cys est le résidu de cystéine de la protéine native sous la forme d'acide cystéique déterminé lors de l'analyse,

et n.d. signifie non déterminé,

les autres acides aminés étant désignés conformément aux recommandations de l'IUPAC,

et dont la séquence amino-terminale est :

```
                                      10                16
Asp-Val-Ser-Phe-Ser-Leu-Ser-Gly-Gly-Gly-Thr-Ala-Ser-Tyr-Glu-Lys
```

La composition en acides aminés a été déterminée par chauffage de la glycoprotéine à 105° C pendant 24 heures sous azote en présence d'acide chlorhydrique 6 N, de phénol à 1 % et de 2-mercaptoéthanol à 1 %. Les acides aminés ont été dérivés par le phénylisothiocyanate (PITC) en milieu alcalin. Les phénylthiohydantoïnes (PTH) ainsi obtenus ont été analysés par HPLC en phase inverse. L'acide cystéique a été déterminé après oxydation de la protéine avec l'acide performique (J. Biol. Chem. 1963, 238, 235-237).

La séquence amino-terminale a été déterminée sur un microséquenceur commercialisé par Applied Biosynthesis à partir de 5 nmoles de protéines solubilisées dans une solution aqueuse d'acide acétique à 10 %. Les PTH des aminoacides sont analysés par HPLC en phase inverse.

Tous ces résultats comparés avec ceux publiés dans la littérature pour la trichosanthine indiquent clairement que la trichokirine et la trichosanthine sont 2 substances totalement différentes.

Selon un autre de ses aspects, la présente invention concerne un procédé pour l'obtention de la trichokirine caractérisé en ce que l'on extrait les graine broyées de Trichosanthes kirilowii avec de l'eau en présence d'un tampon à pH 6,5 - 7,5, on élimine le matériau insoluble et on fractionne l'extrait par chromatographie sur une résine échangeuse d'ions faiblement acide, en éluant avec une solution tampon contenant du chlorure de sodium 0 - 0,6 M à un pH de 7,2 - 7,7, on isole la fraction contenant la protéine inhibitrice, et on la purifie, les produits de faibles poids moléculaires peuvent être éliminés par dialyse de l'extrait.

Par exemple, les graines de Trichosanthes kirilowii, de préférence après décorticage, sont broyées et extraites avec de l'eau contenant un électrolyte par exemple du chlorure de sodium, et un tampon, par exemple à base de phosphate, à un pH voisin de la neutralité. Les matières insolubles sont ensuites éliminées par exemple par centrifugation et les substances de faibles poids moléculaires sont éliminées de la solution par dialyse par exemple contre du tampon phosphate. L'extrait dialysé est alors soumis à un fractionnement, par exemple par chromatographie sur une résine échangeuse d'ions faiblement acide telle que la carboxyméthylcellulose. L'élution, lorsqu'on utilise cette résine, peut être effectuée en utilisant un gradient linéaire de concentration, par exemple une solution aqueuse de 0 à 0,3 M de chlorure de sodium. Normalement, 4 pics principaux sont élués et le 4ème pic élué par une solution de concentration en chlorure de sodium de 0,03 à 0,11 M contient la plus grande partie de la trichokirine.

La purification de la trichokirine peut être achevée par filtration sur gel, par exemple sur une colonne de Sephadex (marque déposée).

La trichokirine peut être couplée aux haptomères par l'intermédiaire d'un agent de couplage approprié. Ainsi, par exemple, la trichokirine peut être couplée à un anticorps par un pont disulfure selon des méthodes classiques et former ainsi une immunotoxine.

Pour cela, aussi bien la trichokirine que l'haptomère peuvent être soumis à une réaction avec un réactif permettant d'introduire une chaîne portant un groupe disulfure.

Un autre objet de l'invention est constitué par les dérivés modifiés de trichokirine contenant un groupe SH libre ou bloqué et plus particulièrement une S-acétylmercaptosuccinoyl-trichokirine obtenue par traitement de la trichokirine par l'anhydride S-acétylmercaptosuccinique, ainsi que les dérivés mercaptosuccinoyle correspondants préparés par action de l'hydroxylamine sur le dérivé S-acétyle. La méthode de préparation de la trichokirine ainsi modifiée est celle qui est couramment utilisée pour la préparation de produits, notamment de protéines, activées.Ainsi, l'introduction du groupe S-acétylmercaptosuccinoyle sur un anticorps est décrite dans le brevet US 4 340 535 ; l'introduction du groupe S-acétylmercaptosuccinoyle sur l'hydrolyse primaire d'un ionophore est décrite dans la demande de brevet européen 162 781 et l'introduction du groupe S-acétylmercaptosuccinoyle sur la chaîne A de la ricine est décrite dans la demande de brevet européen 169 111.

Ces trichokirines modifiées peuvent être représentées par la formule :

$$TK^{\circ} - (CO - \underset{\displaystyle CH_2COOH}{\underset{|}{CH}} - S - W)_n \qquad (I)$$

dans laquelle $TK^0$ représente le radical de la trichokirine, W représente l'hydrogène ou le groupe acétyle et n peut varier entre 0,2 et 3, de préférence entre 0,4 et 1, de préférence encore n = 0,7.

La trichokirine de la présente invention inhibe les synthèses protéiques dans un lysat de réticulocytes avec une $DI_{50}$ de 1,5 - 4 ng/ml. Sur les cellules intactes, l'effet est de loin inférieur, avec une $DI_{50}$ qui peut varier de 7 à plus de 100 µg/ml, selon la cellule examinée.

Le tableau I montre l'effet de la trichokirine sur diverses cellules, notamment la $DI_{50}$ (en µg/ml et en $M.10^{-7}$) de l'inhibition de la synthèse protéique ; celle-ci a été déterminée en appliquant la méthode décrite dans J. Biol. Chem. (1984) 259 p. 9359-9364.

TABLEAU I

| Cellule | $DI_{50}$ µg/ml | $M.10^{-7}$ |
|---|---|---|
| Fibroblastes humains | 3,9 | 1,3 |
| Cellules TG | 7,5 | 2,5 |
| Cellules NB 100 (neuroblastome) | 10,3 | 3,4 |
| Cellules JAR (choriocarcinome) | 16,3 | 5,4 |
| Cellules HeLa | 45,0 | 15,0 |
| M 4039 (melanome) | 9,9 | 3,3 |

La toxicité de la trichokirine chez l'animal a été évaluée sur des souris femelles Swiss de poids 27-32 g. La trichokirine est administrée par voie intrapéritonéale à des doses allant de 0,56 à 20 mg/kg de poids corporel (avec un rapport 2 entre 2 doses) à des groupes de 6 animaux par dose. La dose léthale 50 ($DL_{50}$) est calculée par la méthode de Spearman-Karber telle que décrite par Finney (Statistical Methods in Biological Assay, p 524-530). La $DL_{50}$ de la trichokirine est de 8,1 mg/kg ± 20 %.

Les exemples suivants illustrent l'invention.

EXEMPLE 1 : ISOLEMENT DE LA TRICHOKIRINE

A) Extrait brut

Des graines de Trichosanthes kirilowii (1,2 kg) sont broyées dans un appareil Ultraturrax avec 8 volumes de solution aqueuse de chlorure de sodium 0,14 M contenant du tampon phosphate 5 mM, pH 7,5. L'extraction est poursuivie pendant une nuit à 4° C sous agitation magnétique.

Après élimination des résidus grossiers, on procède à une centrifugation à 10.000 g à 0° C pendant 45 minutes. Le surnageant est décanté, à froid pour éliminer les graisses solidifiées puis les protéines sont précipitées par addition de sulfate d'ammonium jusqu'à saturation. Après centrifugation comme indiqué ci-dessus, le produit précipité est remis en suspension dans 1 litre de solution aqueuse 0,14 M de chlorure de sodium contenant du tampon phosphate 5 mM, pH 7,5. Le produit non dissous est éliminé par centrifugation dans les mêmes conditions que ci-dessus. Le surnageant est passé sur une colonne de Sephadex RG 25 coarse commercialisée par Pharmacia (95 cm x 10 cm) équilibrée avec due tampon phosphate 5 mM, pH 7,0 à température ambiante et élué à la vitesse de 3 l/heure. Le premier pic protéique est collecté ; c'est l'extrait brut.

B) Chromatographie d'échange d'ions

Les extraits bruts combinés de 2 opérations (provenant à l'origine de 2,4 kg de graines) sont additionnés de chlorure de sodium jusqu'à une concentration finale de 30 mM et passés sur une colonne (17,5 cm x 5 cm) de CM-Sepharose RFast Flow commercialisée par Pharmacia, équilibrée avec du tampon phosphate 30 mM,pH 7,0. Après lavage de la colonne avec le même tampon, la protéine liée est éluée par un gradient contenant de 30 à 110 mM de chlorure de sodium dans le même tampon phosphate (volume 20 litres). Des fractions de 400 ml sont collectées à la vitesse de 1,2 litres par heure. On suit l'élution en mesurant l'absorbance à 280 nm et la conductibilité de l'éluat. Les fractions montrant une activité inhibitrice de la synthèse protéique sont réunies (3,6 l) et amenées à pH 5,8 par de l'acide acétique. On passe la

solution sur une colonne de S-Sepharose Fast Flow (6,6 cm x 5 cm) équilibrée avec le tampon acétate de sodium 10 mM, pH 4,5. Après lavage de la colonne avec le même tampon, la protéine inhibitrice fixée est éluée par du tampon phosphate 20 mM, pH 7,5 contenant du chlorure de sodium 0,5 M.

La fraction correspondant au pic contenant la protéine inhibitrice (200 ml) est recueillie et dialysée contre 3 fois 50 l d'eau distillée puis lyophilisée. On obtient ainsi 380 mg de trichokirine brute.

C) Filtration sur gel

La trichokirine obtenue ci-dessus est dissoute dans 50ml de solution aqueuse de chlorure de sodium 0,14 M. On élimine un insoluble par centrifugation à 20.000 g pendant 30 minutes. Le surnageant est passé sur une colonne de Séphadex G 50 coarse (95 cm x 5 cm) équilibrée avec un tampon phosphate 30 mM, pH 7 à 4 °C. On élue à la vitesse de 60 ml/heure. La fraction correspondant au pic contenant la protéine est collectée, dialysée et lyophilisée comme indiqué ci-dessus.

On obtient ainsi 267 mg de trichokirine purifiée.

EXEMPLE 2 : S-acétylmercaptosuccinoyl-trichokirine et Mercaptosuccinoyl-trichokirine

A 5,8 mg de trichokirine (0,215 umole environ) dissoute dans 6 ml de tampon phosphate 125 mM pH7 (soit 0,973 mg/ml, 0,036 µmole environ/ml) sont additionnés 100 µlitres d'une solution d'anhydride S-acétylmercaptosuccinique (SAMSA) à 10,5 mg/ml (60,33 µmoles/ml) dans le diméthylformamide. L'incubation dure une heure puis le milieu réactionnel est purifié de l'excès de réactif par dialyse contre du tampon phosphate 125 mM pH 7. On obtient ainsi 5,7 ml d'une solution de S-acétylmercaptosuccinoyl-trichokirine à 0,948 mg/ml. Par action de l'hydroxylamine et selon la méthode d'Ellman (Methods in Enzymology 25, 457 (1972)), on obtient du mercaptosuccinoyl-trichokirine, ou trichokirine activée, contenant 0,7 groupes SH libres par mole de trichokirine (dosage spectrophotométrique). Examinée par électrophorèse en gradient de polyacrylamide, cette protéine modifiée présente une seule bande de poids moléculaire voisin de 28.000 ± 3.000 identique à celle de la protéine native.

EXEMPLE 3 : Conjugué obtenu par réaction entre l'anticorps AT15E (Anti-thy 1.2) et la mercaptosuccinoyl-trichokirine

A) Préparation de l'anticorps AT15E modifié.

L'anticorps AT15E est un anticorps monoclonal dirigé contre l'antigène Thy 1.2 des lymphocytes murins. Cet anticorps est celui qui est décrit dans Journal of Immunology 122, 2491-8 (1979) et a été obtenu à partir de l'hybridome décrit dans Hybridoma 1 (1) ; 13-17 (1981).

A 3,7 ml d'une solution d'anticorps AT15E à 3,55 mg/ml (soit 0,087 µmole) dans un tampon borate 0,1 M pH 8,8 est ajoutée 25 microlitres d'une solution de N-succinimidyl 3-(pyridyl-2-disulfanyl)-3 propionate à 5,5 mg/ml dans l'éthanol.

L'incubation dure 30 minutes puis l'excès de réactif est éliminé par dialyse contre du tampon phosphate 125 mM pH 7. Après dialyse, la solution protéique est centrifugée et l'on obtient 3,3 ml d'une solution à 3,4 mg/ml. Par dosage spectrophotométrique à 343 nm de la pyridine thione-2 libérée par échange avec le mercapto-2 éthanol on constate que l'on a obtenu un anticorps activé portant 3,2 groupements disulfure mixtes par mole d'anticorps.

B) Préparation de l'immunotoxine (conjugé)

A 1,9 ml de la solution d'anticorps activé précédemment obtenue (soit 0,043 µmole), on ajoute 5 ml de la solution de trichokirine modifiée précédemment obtenue (soit 0,183 µmole) et on incube 20 heures à 25 °C en présence de 350 µlitres d'une solution aqueuse molaire de chlorhydrate d'hydroxylamine. La solution est centrifugée puis purifiée par filtration sur colonne de Sephadex G 200 avec mesure de la densité optique de l'effluent à 280 nm. Le regroupement des fractions contenant à la fois l'anticorps et la TK conduit à 20 ml d'une solution d'immunotoxine à 0,215 mg/ml (soit 4,3 mg). Cette solution contient 0,045 mg/ml de trichokirine modifiée couplée à l'anticorps.

Le taux de couplage moyen dans cette préparation est de 1,5 TK par mole d'anticorps.

Tests d'activité des immunotoxines

L'une des propriétés de la trichokirine est d'inhiber la synthèse des protéines dans les cellules eucaryotes. Les tests réalisés sont donc des tests d'inhibition de la synthèse protéique :

- soit sur un modèle acellulaire,
- soit sur un modèle cellulaire.

A) le modèle acellulaire : activité inhibitrice de la toxine.

Le protocole in vitro utilise des fractions subcellulaires de foie de rat convenablement complémentées et capables d'incorporer la 14C-phénylalanine en présence d'un ARN messager artificiel, l'acide polyuridylique.

Le mode opératoire employé pour préparer les fractions subcellulaires et pour mesurer l'incorporation de 14C-phénylalanine est une adaptation de la méthode décrite dans BBA 312, 608-615 (1973), mettant en oeuvre à la fois une faction microsomale et une fraction de cytosol des hépatocytes de rat. L'échantillon contenant la trichokirine est introduit sous la forme d'une solution convenablement diluée dans un tampon tris-HCl 50 mM pH 7,6 contenant du mercapto-2 éthanol à 0,2 % et de la sérum albumine bovine à 15 µgrammes/ml. A partir des données de comptage on calcule, par rapport à un milieu témoin sans inhibiteur, le pourcentage d'inhibition de l'incorporation de 14C-phénylalanine pour chaque milieu réactionnel.

L'ensemble des valeurs obtenues permet de déterminer la concentration de trichokirine (ou $CI_{50}$) qui inhibe 50 % de l'incorporation de la 14C-phénylalanine dans les conditions de l'essai. Pour la trichokirine native, on a ainsi déterminé que la $CI_{50}$ est égale à $7.10^{-11}$M, et que celle de la trichokirine modifiée est égale à 8 - $10.10^{-11}$ mole/litre. La modification n'entraîne donc pas de perte importante d'activité de la trichokirine.

B) Cytotoxicité spécifique de l'immunotoxine à trichokirine sur modèle cellulaire.

L'activité biologique de la trichokirine est d'inhiber la synthèse protéique cellulaire de systèmes eucaryotes. La technique utilisée met en oeuvre en modèle cellulaire dans lequel on mesure l'effet des substances étudiées sur l'incorporation de 14C-leucine dans les cellules cancéreuses en culture. Les cellules utilisées appartiennent à la lignée cellulaire T2, issue d'une leucémie T de souris qui exprime l'antigène de surface Thy 1.2. Les cellules sont incubées en présence de la substance à étudier à différentes concentrations puis, en fin d'incubation, on procède à la mesure du taux d'incorporation de 14C-leucine par les cellules ainsi traitées.

Cette mesure est effectuée selon une technique adaptée de la procédure décrite dans Journal of Biological Chemistry 1974, 249 (II), 3557-3562, utilisant le traceur 14C-leucine pour la détermination du taux de synthèse protéique. La détermination de la radioactivité incorporée est effectuée ici sur les cellules entières isolées par filtration.

A partir de ces déterminations, on peut tracer les courbes effets doses présentant en abscisse la concentration molaire en trichokirine des substances étudiées et en ordonnée l'incorporation de 14C-leucine exprimée en pourcentage de l'incorporation par les cellules témoins cultivées en l'absence de toute substance affectant la synthèse protéique.

On peut ainsi déterminer pour chaque substance étudiée la concentration qui inhibe 50 % de l'incorporation de 14C-leucine ou "concentration inhibitrice 50" ($CI_{50}$). Le tableau 2 représente les valeurs de $CI_{50}$ obtenues dans la même expérience avec l'immunotoxine à trichokirine d'une part, la trichokirine non couplée d'autre part.

TABLEAU 2

| Produits | Concentration inhibitrice 50 |
|---|---|
| IT à trichokirine | 2 x $10^{-10}$ M |
| Trichokirine | 2 x $16^{-6}$ M |

On peut constater que l'IT à trichokirine a une très forte activité cytotoxique qui est plus de 10.000 fois supérieure à celle de la trichokirine non couplée.

Cette nouvelle classe d'immunotoxines peut être utilisée pour le traitement des affections cancéreuses ou non, pour lesquelles les cellules à détruire seraient reconnues par l'anticorps utilisé pour la préparation de l'immunotoxine. Les modalités optimales d'administration ainsi que la durée du traitement devront être déterminées dans chaque cas en fonction du sujet et de la nature de l'affection à traiter.

Les produits biologiquement actifs de l'invention peuvent être formulés pour l'administration par toute voie convenable. Le plus souvent, le principe actif sera administré par injection et il sera formulé dans un liquide stérile apyrogène, de préférence de l'eau qui peut contenir des sels physiologiquement acceptables tels que des tampons ou du chlorure de sodium de façon à obtenir une solution isotonique.

Pour un traitement antitumoral, la dose journalière de trichokirine conjuguée à un anticorps, est de préférence comprise entre 1 et 100 mg, avantageusement de 5 à 50 mg et par exemple de 10 mg environ.

Les unités de dosage contenant la trichokirine sous forme de conjugué, par exemple des ampoules pour injection, contiendront donc habituellement de 1 à 100 mg de conjugué de trichokirine.

**Revendications**

**Revendications pour les Etats contractants suivants : AT, BE, CH, DE, FR, GB, GR, IT, LI, LU, NL, SE**

**1.** Protéine inhibant les ribosomes, nommée trichokirine, ayant les caractéristiques suivantes : une glycoprotéine :
   - ayant un poids moléculaire de 28.000 ± 3.000 déterminé par électrophorèse sur gel de polyacrylamide en présence de dodécylsulfate de sodium ;
   - de point isoélectrique ≧ 9 ;
   - de contenu en sucres neutres de 1,1 à 1,5 % en poids dont 0,3 à 1,2 % de mannose ;
   - de compositions en acides aminés, en nombre de résidus/mole de protéine avec un écart en plus ou en moins de 20 % :
     Lys : 17,3 ; His : 1,1 ; Arg : 6,7 ; Asx : 22,8 ;
     Thr : 18,9 ; Ser : 23,5 ; Glx : 21,6 ; Pro : 8,0 ;
     Gly : 16,0 ; Ala : 21,1 ; 1/2 Cys : 1,9 ;
     Val : 12,5 ; Met : 3,05 ; Ile : 15,8 ; Leu : 24,3 ;
     Tyr : 12,1 ; Phe : 10,1 ; Trp : n.d..

   telle que Asx est l'ensemble de résidus d'acide aspartique et d'asparagine
   Glx est l'ensemble de résidus d'acide glutamique et de glutamine,
   1/2 Cys est le résidu de cystéine de la protéine native sous la forme d'acide cystéique déterminé lors de l'analyse,
   et n.d. signifie non déterminé,
   les autres acides aminés étant désignés conformément aux recommandations de l'IUPAC,
   et dont la séquence amino-terminale est :

1 10 16

Asp-Val-Ser-Phe-Ser-Leu-Ser-Gly-Gly-Gly-Thr-Ala-Ser-Tyr-Glu-Lys.

2. Dérivés mercaptosuccinoyl-trichokirine de formule :

$$TK° - (CO - \underset{\displaystyle CH_2COOH}{\underset{|}{CH}} - S - W)_n$$

dans laquelle TK° représente le radical de la trichokirine, W représente l'hydrogène ou le groupe acétyle et n peut varier entre 0,2 et 3.

3. Dérivés selon la revendication 2, caractérisés en ce que, dans sa formule, n varie entre 0,4 et 1.

4. Dérivés selon l'une des revendications 2 ou 3, caractérisés en ce que, dans sa formule, W est l'hydrogène et n est 0,7.

5. Dérivés selon l'une des revendications 2 ou 3, caractérisés en ce que, dans sa formule, W est acétyle et n est 0,7.

6. Procédé pour la préparation de la trichokirine, selon la revendication 1, caractérisé en ce que l'on extrait des graines broyées de Trichosanthes kirilowii par une solution aqueuse tamponnée à pH 6,5 - 7,5, on élimine le matériau insoluble et on fractionne l'extrait brut par chromatographie sur une résine échangeuse d'ions faiblement acide, en éluant avec une solution tampon contenant du chlorure de sodium à un pH de 7,2 - 7,7, et on récupère la fraction protéique inhibitrice et purifie la protéine par filtration sur gel.

7. Procédé selon la revendication 6, caractérisé en ce qu'on élimine de l'extrait par dialyse les produits de faible masse moléculaire.

8. Procédé pour la préparation de dérivés de trichokirine décrits dans l'une quelconque des revendications 2 à 5, caractérisé en ce que l'on fait réagir sur la trichokirine, l'anhydride S-acétylmercaptosuccinique puis éventuellement on fait réagir sur le dérivé S-acétyle ainsi obtenu l'hydroxylamine.

9. Utilisation de la trichokirine ou de ses dérivés selon l'une quelconque des revendications 1 à 5 pour la préparation de conjugués par couplage avec des haptomères.

**Revendications pour l'Etat contractant suivant : ES**

1. Procédé pour l'obtention d'une protéine inhibant les ribosomes, nommée trichokirine, ayant les caractéristiques suivantes : c'est une Glycoprotéine :
   - ayant un poids moléculaire de 28.000 ± 3.000 déterminé par électrophorèse sur gel de polyacrylamide en présence de dodécylsulfate de sodium ;
   - de point isoélectrique ≧ 9 ;
   - de contenu en sucres neutres de 1,1 à 1,5 % en poids dont 0,3 à 1,2 % de mannose ;
   - de compositions en acides aminés, en nombre de résidus/mole de protéine avec un écart en plus ou en moins de 20 % :
     Lys : 17,3 ; His : 1,1 ; Arg : 6,7 ; Axs : 22,8 ;
     Thr : 18,9 ; Ser : 23,5 ; Glx : 21,6 ; Pro : 8,0 ;
     Gly : 16,0 ; Ala : 21,1 ; 1/2 Cys : 1,9 ;
     Val : 12,5 ; Met : 3,05 ; Ile : 15,8 ; Leu : 24,3 ;
     Tyr : 12,1 ; Phe : 10,1 ; Trp : n.d..

telle que Asx est l'ensemble de résidus d'acide aspartique et d'asparagine
Glx est l'ensemble de résidus d'acide glutamique et de glutamine,

1/2 Cys est le résidu de cystéine de la protéine native sous la forme d'acide cystéique déterminé lors de l'analyse, et n.d. signifie non déterminé,
les autres acides aminés étant désignés conformément aux recommandations de l'IUPAC,
et dont la séquence amino-terminale est :

1 10 16
Asp-Val-Ser-Phe-Ser-Leu-Ser-Gly-Gly-Gly-Thr-Ala-Ser-Tyr-Glu-Lys,

caractérisé en ce que l'on extrait des graines de Trichosanthes kirilowii par une solution aqueuse tamponnée à pH 6,5 - 7,5, on élimine le matériau insoluble et on fractionne l'extrait brut par chromatographie sur une résine échangeuse d'ions faiblement acide, en éluant avec une solution tampon contenant du chlorure de sodium à un pH de 7,2 - 7,7 et on récupère la fraction protéique inhibitrice et purifie la protéine par filtration sur gel.

2. Procédé selon la revendication 1, caractérisé en ce qu'on élimine de l'extrait par dialyse les produits de faible masse moléculaire.

3. Procédé pour l'obtention de dérivés mercaptosuccinoyle de trichokirine représentés par la formule :

$$TK^{\circ} - (CO - \underset{\displaystyle CH_2COOH}{\underset{|}{CH}} - S - W)_n$$

dans laquelle TK° représente le radical de la trichokirine, W représente l'hydrogène ou le groupe acétyle et n peut varier entre 0,2 et 3, caractérisé en ce que l'on fait réagir sur la trichokirine, obtenue par le procédé selon l'une des revendications 1 ou 2, l'anhydride S-acétylmercaptosuccinique et en ce qu'éventuellement on fait réagir le produit obtenu avec de l'hydroxylamine.

4. Utilisation de la trichokirine ou de ses dérivés obtenus par l'un des procédés selon l'une quelconque des revendications 1 à 3 pour la préparation de conjugués par couplage avec des haptomères.

**Claims**

**Claims for the following Contracting States : AT, BE, CH, DE, FR, GB, GR, IT, LI, LU, NL, SE**

1. Ribosome-inhibiting protein called trichokirin, which has the following characteristics: a glycoprotein
   - having a molecular weight of 28,000 ± 3000 as determined by electrophoresis on polyacrylamide gel in the presence of sodium dodecyl sulfate;
   - with an isoelectric point ≧ 9;
   - with a content of neutral sugars of 1.1 to 1.5% by weight, including 0.3 to 1.2% of mannose;
   - having the following amino acid composition, expressed as the number of residues/mol of protein with a deviation of plus or minus 20%:

     Lys : 17.3 ; His : 1.1 ; Arg : 6.7 ; Asx : 22.8 ;
     Thr : 18.9 ; Ser : 23.5 ; Glx : 21.6 ; Pro : 8.0 ;
     Gly : 16.0 ; Ala : 21.1 ; 1/2 Cys : 1.9 ;
     Val : 12.5 ; Met : 3.05; Ile : 15.8 ; Leu : 24.3 ;
     Tyr : 12.1 ; Phe : 10.1 ; Trp : n.d.

     where Asx is the aspartic acid and asparagine residues together,
     Glx is the glutamic acid and glutamine residues together,
     1/2 Cys is the cystein residue of the native protein in cysteic acid form determined during the analysis and n.d. means "not determined",
     the other amino acids being designated according to the IUPAC recommendations ; and
   - having the following terminal amino sequence:

1 10
Asp-Val-Ser-Phe-Ser-Leu-Ser-Gly-Gly-Gly-
16
Thr-Ala-Ser-Tyr-Glu-Lys.

2. Trichokirin mercaptosuccinoyl derivatives of the formula:

$$TK^{\circ} - (CO-\underset{\underset{CH_2COOH}{|}}{CH}-S-W)_n$$

in which TK° represents the radical of trichokirin, W represents hydrogen or the acetyl group and n can vary between 0.2 and 3.

3. Derivatives according to claim 2, characterized in that in its formula, n varies between 0.4 and 1.

4. Derivatives according to one of claims 2 or 3, characterized in that in its formula, W is hydrogen and n is 0.7.

5. Derivatives according to one of claims 2 or 3, characterized in that in its formula, W is acetyl and n is 0.7.

6. Process for the preparation of trichokirin according to claim 1, characterized in that it comprises extracting ground seeds of Trichosanthes kirilowii with an aqueous solution buffered at pH 6.5-7.5, removing the insoluble material, fractionating the crude extract by chromatography on a slightly acidic ion exchange resin, using as eluent a buffer solution containing sodium chloride at a pH of 7.2-7.7 and recovering the inhibitor protein fraction and purifying the protein by gel filtration.

7. Process according to claim 6, characterized in that the products of low-molecular mass are removed from the extract by dialysis.

8. Process for the preparation of trichokirin derivatives as described in any one of claims 2 to 5, characterized in that it comprises reacting S-acetylmercaptosuccinic anhydride with trichokirin, and if appropriate, reacting the hydroxylamine on the resulting S-acetyl derivative.

9. Use of the trichokirin or of one of its derivatives according to any one of claims 1 to 5 for the preparation of conjugates by coupling with haptomers.

**Claims for the following Contracting State : ES**

1. Process for the preparation of a ribosome-inhibiting protein called trichokirin, which has the following characteristics: a glycoprotein
   - having a molecular weight of 28,000 ± 3000 as determined by electrophoresis on polyacrylamide gel in the presence of sodium dodecyl sulfate;
   - with an isoelectric point ≧ 9;
   - with a content of neutral sugars of 1.1 to 1.5% by weight, including 0.3 to 1.2% of mannose;
   - having the following amino acid composition, expressed as the number of residues/mol of protein with a deviation of plus or minus 20%:

   Lys : 17.3 ; His : 1.1 ; Arg : 6.7 ; Asx : 22.8 ;
   Thr : 18.9 ; Ser : 23.5 ; Glx : 21.6 ; Pro : 8.0 ;

Gly : 16.0 ; Ala : 21.1 ; 1/2 Cys : 1.9 ;
Val : 12.5 ; Met : 3.05; Lle : 15.8 ; Leu : 24.3 ;
Tyr : 12.1 ; Phe : 10.1 ; Trp : n.d.

where Asx is the aspartic acid and asparagine residues together,
Glx is the glutamic acid and glutamine residues together,
1/2 Cys is the cystein residue of the native protein in cysteic acid form determined during the analysis and n.d. means "not determined",
the other amino acids being designated according to the IUPAC recommendations ; and

- having the following terminal amino sequence:

```
1                                      10
Asp-Val-Ser-Phe-Ser-Leu-Ser-Gly-Gly-Gly-
                        16
Thr-Ala-Ser-Tyr-Glu-Lys,
```

characterized in that it comprises extracting ground seeds of Trichosanthes kirilowii with an aqueous solution buffered at pH 6.5-7.5, removing the insoluble material, fractionating the crude extract by chromatography on a slightly acidic ion exchange resin, using as eluent a buffer solution containing sodium chloride at a pH of 7.2-7.7 and recovering the inhibitor protein fraction and purifying the protein by gel filtration.

2. Process according to claim 1, characterized in that the products of low-molecular mass are removed from the extract by dialysis.

3. Process for the preparation of trichokirin mercaptosuccinoyl derivatives of the formula:

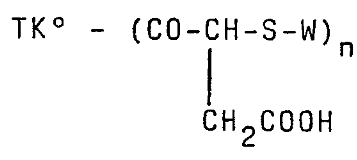

in which TK° represents the radical of trichokirin, W represents hydrogen or the acetyl group and n can vary between 0.2 and 3, characterized in that it comprises reacting the S-acetylmercaptosuccinic anhydride with the trichokirin obtained by the process according to one of claims 1 or 2, and, if appropriate, reacting the resulting product with hydroxylamine.

4. Use of the trichokirin or of one of its derivatives obtained by one of the processes according to any one of claims 1 to 3, for the preparation of conjugates by coupling with haptomers.

**Patentansprüche**
**Patentansprüche für folgende Vertragsstaaten : AT, BE, CH, DE, FR, GB, GR, IT, LI, LU, NL, SE**

1. Ribosomeninhibierendes, als Trichokirin bezeichnetes Protein, das ein Glykoprotein mit folgenden Eigenschaften darstellt:

   - Es besitzt ein durch Gelelektrophorese an Polyacrylamid in Gegenwart von Natriumdodecylsulfonat bestimmtes Molekulargewicht von 28.000 ± 3.000;
   - der isoelektrische Punkt ist ≧ 9;
   - der Gehalt an neutralen Zuckern beträgt 1,1 bis 1,5 Gew.-%, wovon 0,3 bis 1,2 % Mannose sind;
   - es weist folgende Aminosäurezusammensetzung auf, ausgedrückt als Anzahl der Reste/Mol Protein bei einer Genauigkeit von ± 20 %:

Lys: 17,3; His: 1,1; Arg: 6,7; Asx: 22,8;
Thr: 18,9; Ser: 23,5; Glx: 21,6; Pro: 8,0;
Gly: 16,0 Ala: 21,1; 1/2 Cys: 1,9;
Val: 12,5; Met: 3,05 Ile: 15,8; Leu: 24,3;
Tyr: 12,1; Phe: 10,1; Trp: nicht bestimmt,

wobei

Asx die Gesamtheit der Asparaginsäure- und Asparaginreste,

Glx die Gesamtheit der Glutaminsäure- und Glutaminreste bedeuten, und

1/2 Cys den Cysteinrest des nativen Proteins in Form der bei der Analyse bestimmten Cysteinsäure darstellt

und

die Bezeichnung der bestimmten Aminosäuren den IUPAC-Empfehlungen entspricht;

- es besitzt folgende aminoterminale Sequenz:
Asp-Val-Ser-Phe-Ser-Leu-Ser-Gly-Gly-Gly-Thr-Ala-Ser-Tyr-Glu-Lys.

**2.** Mercaptosuccinoyl-trichokirinderivate der Formel

$$TK^\circ - (CO - \underset{\displaystyle CH_2COOH}{\overset{}{CH}} - S - W)_n \quad ,$$

in der bedeuten:

TK° den Rest des Trichokirins,

W Wasserstoff oder Acetyl und

n eine Zahl zwischen 0,2 und 3.

**3.** Derivate nach Anspruch 2, dadurch gekennzeichnet, daß n in der Formel eine Zahl zwischen 0,4 und 1 bedeutet.

**4.** Derivate nach Anspruch 2 oder 3, dadurch gekennzeichnet, daß in der Formel W Wasserstoff und n 0,7 bedeuten.

**5.** Derivate nach Anspruch 2 oder 3, dadurch gekennzeichnet, daß in der Formel W Acetyl und n 0,7 bedeuten.

**6.** Verfahren zur Herstellung des Trichokirins nach Anspruch 1,

gekennzeichnet durch

- Extraktion zerkleinerter Samen von Trichosanthes kirilowii mit einer wäßrigen, auf pH 6,5 - 7,5 gepufferten Lösung,
- Abtrennung des unlöslichen Materials,
- Fraktionierung des Rohextrakts durch Chromatographie an einem schwach sauren Ionenaustauscherharz unter Elution mit einer Natriumchlorid enthaltenden Pufferlösung bei pH 7,2 - 7,7,
- Gewinnung der Proteinfraktion mit Inhibitorwirkung
und
- Reinigung des Proteins durch Gelfiltration.

**7.** Verfahren nach Anspruch 6, dadurch gekennzeichnet, daß die Verbindungen kleiner Molekülmasse durch Dialyse aus dem Extrakt abgetrennt werden.

8. Verfahren zur Herstellung der Trichokirinderivate nach einem der Ansprüche 2 bis 5, gekennzeichnet durch Umsetzung von Trichokirin mit S-Acetylmercaptobernsteinsäureanhydrid sowie ggfs. anschließende Umsetzung des so erhaltenen S-Acetylderivats mit Hydroxylamin.

9. Verwendung des Trichokirins oder seiner Derivate nach einem der Ansprüche 1 bis 5 zur Herstellung von Konjugaten durch Kopplung mit Haptomeren.

**Patentansprüche für folgenden Vertragsstaat : ES**

1. Verfahren zur Herstellung eines ribosomeninhibierenden, als Trichokirin bezeichneten Proteins, das ein Glykoprotein mit folgenden Eigenschaften darstellt:

   - Es besitzt ein durch Gelelektrophorese an Polyacrylamid in Gegenwart von Natriumdodecylsulfonat bestimmtes Molekulargewicht von 28.000 ± 3.000;
   - der isoelektrische Punkt ist ≧ 9;
   - der Gehalt an neutralen Zuckern beträgt 1,1 bis 1,5 Gew.-%, wovon 0,3 bis 1,2 % Mannose sind;
   - es weist folgende Aminosäurezusammensetzung auf, ausgedrückt als Anzahl der Reste/Mol Protein bei einer Genauigkeit von ± 20 %:

     Lys: 17,3; His: 1,1; Arg: 6,7; Asx: 22,8;
     Thr: 18,9; Ser: 23,5; Glx: 21,6; Pro: 8,0;
     Gly: 16,0 Ala: 21,1; 1/2 Cys: 1,9;
     Val: 12,5; Met: 3,05 Ile: 15,8; Leu: 24,3;
     Tyr: 12,1; Phe: 10,1; Trp: nicht bestimmt,

     wobei
     Asx die Gesamtheit der Asparaginsäure- und Asparaginreste,
     Glx die Gesamtheit der Glutaminsäure- und Glutaminreste bedeuten und
     1/2 Cys den Cysteinrest des nativen Proteins in Form der bei der Analyse bestimmten Cysteinsäure darstellt
     und
     die Bezeichnung der bestimmten Aminosäuren den IUPAC-Empfehlungen entspricht;
   - es besitzt folgende aminoterminale Sequenz:
     Asp-Val-Ser-Phe-Ser-Leu-Ser-Gly-Gly-Gly-Thr-Ala-Ser-Tyr-Glu-Lys,

   gekennzeichnet durch

   - Extraktion zerkleinerter Samen von Trichosanthes kirilowii mit einer wäßrigen, auf pH 6,5 - 7,5 gepufferten Lösung,
   - Abtrennung des unlöslichen Materials,
   - Fraktionierung des Rohextrakts durch Chromatographie an einem schwach sauren Ionenaustauscherharz unter Elution mit einer Natriumchlorid enthaltenden Pufferlösung bei pH 7,2 - 7,7,
   - Gewinnung der Proteinfraktion mit Inhibitorwirkung
     und
   - Reinigung des Proteins durch Gelfiltration.

2. Verfahren nach Anspruch 1, dadurch gekennzeichnet, daß die Verbindungen kleiner Molekülmasse durch Dialyse aus dem Extrakt abgetrennt werden.

3. Verfahren zur Herstellung von Mercaptosuccinoylderivaten des Trichokirins der Formel

$$TK^{\circ} - (CO - \underset{\displaystyle CH_2COOH}{\overset{|}{CH}} - S - W)_n ,$$

in der bedeuten:

| | |
|---|---|
| TK° | den Rest des Trichokirins, |
| W | Wasserstoff oder Acetyl und |
| n | eine Zahl zwischen 0,2 und 3, |

gekennzeichnet durch Umsetzung des nach dem Verfahren von Anspruch 1 oder 2 erhaltenen Trichokirins mit S-Acetylmercaptobernsteinsäureanhydrid sowie ggfs. anschließende Umsetzung des erhaltenen Produkts mit Hydroxylamin.

**4.** Verwendung des Trichokirins oder seiner Derivate, die nach einem der Verfahren nach einem der Ansprüche 1 bis 3 erhalten wurden, zur Herstellung von Konjugaten durch Kopplung mit Haptomeren.